# EUROPÄISCHE PATENTSCHRIFT

(11) **EP 0 409 078 B1**
(45) Veröffentlichungstag und Bekanntmachung des Hinweises auf die Patenterteilung: **09.11.1994**
(21) Anmeldenummer: 90113328.0
(22) Anmeldetag: 12.07.1990
(51) Int. Cl.: C12Q 1/68

(54) **Verfahren zur Sequenzierung von Desoxyribonukleinsäuren**
Method for sequencing of deoxyribonucleic acid
Procédé de séquence d'acides déoxyribonucléiques

(30) Priorität: 19.07.1989 DE 3923895
(43) Veröffentlichungstag der Anmeldung: 23.01.1991
(73) Patentinhaber: BASF Aktiengesellschaft, 67063 Ludwigshafen (DE)
(72) Erfinder: Wozny, Manfred, Dr., D-6800 Mannheim 51 (DE)

(56) Entgegenhaltungen:
- WO-A-90/04649
- BIOTECHNIQUES, Band 7, 1989, Natick, MA (US); LEVADAKOU et al., Seiten 438-442#
- BIOTECHNIQUES, Band 7, 1989; A.M. CAROTHERS et al., Seiten 494-499#
- NUCLEIC ACIDS RESEARCH, Band 16, Nr. 21, 1988, IRL Press Ltd., Oxford (GB); K.L. NAKAMAYE et al., Seiten 9947-9959#

## Beschreibung

Die Analyse der Nukleotidsequenz Von Nukleinsäuren stellt eine zentrale Technik in der Molekularbiologie dar.

Derzeit stehen zwei grundlegende Techniken für die routinemäßige Sequenzierung von DNA zur Verfügung. Es handelt sich dabei zum einen um die Methode von Maxam und Gilbert. (Methods Enzymol. 65 (1980), 499 - 560), und zum anderen um die Methode von Sanger (Proc. Natl. Acad. Sci. USA 74 (1977), 5463).

Zur Zeit wird zur DNA-Sequenzierung fast ausschließlich die Sanger-Sequenzierung eingesetzt, bei der ausgehend von einem Oligonukleotidprimer, dessen Sequenz zu einem Teil des zu sequenzierenden DNA-Stranges komplementär ist, in Anwesenheit eines Gemisches aus den vier Desoxyribonukleosidtriphosphaten und einem Didesoxyribonukleosidtriphosphat von einer Polymerase komplementäre Kopien des zu sequenzierenden Stranges erstellt werden. Entscheidend ist dabei die Verwendung der Didesoxyribonukleosidtriphosphate, da nach deren Einbau in die neusynthetisierte DNA auf Grund einer fehlenden Hydroxylgruppe eine Kettenverlängerung nicht mehr möglich ist und sich auf diese Weise sequenzspezifische Kettenabbrüche erzeugen lassen. Die Kettenverlängerungsreaktionen werden in der Regel in getrennten Ansätzen für alle vier möglichen Mischungen aus den vier Desoxyribonukleosidtriphosphaten und einem Didesoxyribonukleosidtriphosphat durchgeführt. Anschließend erfolgt eine längenmäßige Auftrennung der erzeugten Nukleinsäurefragmente mit üblichen elektrophoretischen Methoden, die ein direktes Ablesen der Nukleotidsequenz ermöglichen. Zur Unterscheidung der neu synthetisierten DNA von der zu sequenzierenden DNA werden entweder der Oligonukleotidprimer, wenigstens eines der vier Desoxynukleotide oder die Didesoxynukleotide mit z.B. Radioisotopen oder neuerdings auch Fluoreszenzfarbstoffen markiert.

Die Durchführung der Reaktionen erfolgt bei allen derzeit gebräuchlichen Verfahren zwei- bis dreistufig: In einem ersten (bei Einzelstrang-DNA nicht nötigen) Schritt wird eine Trennung der beiden komplementären Stränge der zu sequenzierenden DNA mittels erhöhtem pH oder Hitze vorgenommen. Danach erfolgt zu Beginn der sog. Annealingreaktion bei erhöhter Temperatur (etwa 65°C) die Zugabe des Oligonukleotidprimers, der dann im Verlaufe des Abkühlens der Lösung an den zu sequenzierenden Strang bindet. Im dritten Schritt erfolgt bei einer für die verwendete Polymerase geeigneten Temperatur die eigentliche Sequenzierungsreaktion.

Zur Zeit werden praktisch ausschließlich drei verschiedene Typen von Polymerasen zur Sequenzierung von DNA eingesetzt: Das Klenow-Fragment der E. Coli-Polymerase I, modifizierte Formen der T7-Polymerase und die hitzestabile Taq-Polymerase aus Thermus aquaticus.

Mit den momentan verfügbaren Methoden können alle einzelsträngigen DNA-Proben sowie in der supercoil-Form vorliegende doppelsträngige DNA-Proben routinemäßig sequenziert werden, sofern die letzteren eine kritische Größe (etwa 20 Kilobasen) nicht überschreiten. Eine in der Routine bewährte Vorschrift für die Sequenzierung von supercoil-DNA mit Hilfe der Taq-Polymerase existiert derzeit allerdings nicht. Dieses wäre besonders deswegen wünschenswert, da bei der dabei möglichen höheren Reaktionstemperatur Probleme, die durch die Ausbildung von Sekundärstrukturen verursacht werden, eliminiert werden.

Außer den oben aufgeführten Einschränkungen bereitet vor allem die Sequenzierung von linearen doppelsträngigen DNA-Molekülen Probleme. Derartige DNA-Moleküle treten insbesondere als Endprodukte der sogenannten Polymerase-Chain-Reaction (PCR) (siehe z.B. Mullis et al., Methods Enzymol. 155 (1987), 1973 - 1977) auf. Neben einer breiten Anwendbarkeit in der molekularbiologischen Forschung ist diese hochempfindliche Methode zur in-vitro-Amplifizierung von DNA-Fragmenten vor allem bei der Analyse der molekularen Grundlagen von Erbkrankheiten, bei forensischen Identifikationsproblemen und bei der pränatalen Diagnostik von sehr großer Bedeutung, da in all den genannten Fällen nur geringe Mengen an Nukleinsäure zur Verfügung stehen, die für die Sequenzanalyse nach den gängigen Verfahren nicht ausreichend sind.

Eine Sequenzierungsmethode für PCR-Fragmente, die auf dem Verfahren von Sanger aufbaut, wurde von Saiki et al. (Science 239 (1988), 487 - 491) beschrieben. Diese Methode ist allerdings in hohem Maße von einer optimalen Ausführung der einzelnen aufeinanderfolgenden Reaktionsschritte abhängig, was ihre Anwendbarkeit im Routinebetrieb erschwert. Außerdem wurden noch Verfahren beschrieben, die nach dem Maxam-Gilbert-Prinzip auf einem chemischen Strangbruch aufbauen (z.B. Keohavong et al., DNA 7(1) (1988), 63 - 70; Voss et al., Nucl. Acids, Res. 17(7) (1989), 2517 - 2527). Der Nachteil dieser Verfahren liegt in der Notwendigkeit, daß im Anschluß an die PCR aufwendige chemische Reaktionen mit z.T. toxischen Substanzen durchgeführt werden müssen. Daneben wurde noch über ein prinzipiell sehr elegantes Verfahren berichtet, bei dem schon während der PCR ein sequenzspezifischer Einbau von Thio-desoxyribonukleotiden erfolgt, an denen anschließend mit Hilfe einer Alkylierungsreaktion definierte Strangbrüche erzeugt werden (Nakamaye et al., Nucl. Acids Res. 16(21) (1988), 9947 - 9959). Dieses Verfahren besitzt jedoch den entscheidenden Nachteil, daß damit nur geringe Signalintensitäten erzeugt werden können und der der Sequenzierung zugängliche Bereich auf höchstens 250 Basen beschränkt ist.

In neuester Zeit wurde darüber hinaus noch von Methoden berichtet, bei denen die Taq-Polymerase in einem Thermal Cycler nach der Methodik von Sanger zur Sequenzierung verwendet wird (Levedakou et al., BioTechniques 7 (1989), 438-422; Carothers et al., BioTechniques 7 (1989), 494-499).

Beim Einsatz von Fluoreszenzfarbstoffen zur Markierung der neusynthetisierten Fragmente ist es äußerst wünschenswert, eine Methode zur Verfügung zu haben, mit der eine vielfach höhere Ausbeute an Fragmenten erhalten werden kann als dies mit den herkömmlichen Methoden möglich ist.

Gegenstand der Erfindung ist ein Verfahren zur Sequenzierung von Desoxyribonukleinsäuren, welches darin besteht, daß man die Desoxyribonukleinsäure
a) einem Hitzedenaturierungsschritt
b) einem Annealingschritt und
c) einem Nukleinsäuresyntheseschritt mit teilweisem Kettenabbruch
unterwirft, die Schritte a) bis c) 10- bis 60mal wiederholt und anschließend das erhaltene Desoxyribonukleinsäurefragment-Gemisch der Länge nach auftrennt und aus dem Fragmentspektrum die Sequenz bestimmt.

Die DNA wird den Schritten a) bis c) in einer Konzentration von 10⁻¹⁶ bis 2·10⁻¹⁴ Mol pro µl unterworfen. Im Fall von doppelsträngiger, nicht in der Supercoil-Form vorliegender DNA liegt die Konzentration vorzugsweise im unteren Bereich, das heißt bei etwa 10⁻¹⁶ bis 3·10⁻¹⁵ Mol pro µl.

Der Hitzedenaturierungsschritt erfolgt bei Temperaturen zwischen 92 und 98°C, vorzugsweise 95°C.

Der Annealingschritt gelingt gut bei Temperaturen zwischen 35 und 75°C.

Beim Nukleinsäuresyntheseschritt geht man von einem Oligonukleotidprimer aus, der komplementär zu einem Teilstück des zu sequenzierenden Einzelstrangs sein muß. Dieser Primer, der etwa 10 bis 50, vorzugsweise 15 bis 30 Nukleotide lang ist, wird an die zu analysierende DNA angelagert. Das molare Verhältnis zwischen Oligonukleotidprimer und zu sequenzierender Sequenz beträgt vorzugsweise zwischen 5 und 30, d.h. es werden pro Mol zu sequenzierender Sequenz zwischen 5 und 30 Mol Primer verwendet. Danach wird der Primer in Anwesenheit eines Gemisches aus den vier Desoxyribonukleosidtriphosphaten und mindestens einem Didesoxyribonukleosidtriphosphat komplementär zum zu sequenzierenden DNA-Strang verlängert. Die Verlängerung erfolgt enzymatisch mit einer hitzestabilen Polymerase, z.B. mit der Taq-Polymerase. Der Nukleinsäuresyntheseschritt gelingt im Falle der Taq-Polymerase gut bei einer Temperatur von 65 bis 85°C.

Die Schritte a) bis c) werden etwa 10- bis 60mal, vorzugsweise 10- bis 30mal nacheinander durchgeführt.

Die Zugabe von Didesoxyribonukleosidtriphosphaten dient dazu, die Synthese der komplementären DNA sequenzspezifisch abzubrechen. Zum Kettenabbruch kann man ein bestimmtes der vier Didesoxyribonukleosidtriphosphate verwenden. In diesem Fall muß die DNA-Synthese 4mal, d.h. mit jedem Didesoxyribonukleosidtriphosphat einmal, durchgeführt werden.

Man kann aber auch ein Gemisch der 4 Didesoxyribonukleosidtriphosphate verwenden. In diesem Fall genügt es, die Synthese nur einmal durchzuführen.

Um die neu synthetisierten Nukleinsäuren zu erkennen, müssen diese markiert sein. Das kann durch Einsatz von markiertem Oligonukleotidprimer oder von markierten Desoxy- bzw. Didesoxyribonukleosidtriphosphaten erfolgen. Die Markierung kann mit einem Radioisotop, einem Enzym, einer spezifischen Bindungsstelle für ein detektierbares Molekül oder vorzugsweise mit einem Fluoreszenzfarbstoff erfolgen.

Die Durchführung der oben genannten einzelnen Schritte ist bekannt (z.B. Tabor et al., Proc. Natl. Acad. Sci. USA 84 (1987), 4767-4771).

Das erfindungsgemäße Verfahren besitzt gegenüber den derzeit verfügbaren Verfahren zur Sequenzierung von DNA folgende Vorteile:
a) Es können damit in zuverlässiger und einfacher Weise PCR-Produkte sequenziert werden (Beispiel 1)
b) Das Verfahren ermöglicht die Sequenzierung von hochmolekularen, doppelstrangigen DNA-Proben wie z.B. von Lambda-DNA (Beispiel 2) sowie generell die Sequenzierung von Doppelstrang-DNA, selbst wenn diese nicht in der supercoil-Form vorliegt (Beispiel 3).
c) Es ist auch auf Einzelstrang-DNA anwendbar und führt mit allen DNA-Proben im Vergleich zu herkömmlichen Methoden zu einer vielfach höheren Ausbeute an Reaktionsprodukten, so daß eine entsprechend geringere DNA-Menge für die Sequenzierung ausreichend ist und insbesondere die Anwendbarkeit der Fluoreszenzmarkierung nicht mehr durch die zur Verfügung stehende DNA-Menge eingeschränkt ist.

Von essentieller Bedeutung ist die höhere Fragmentausbeute im Falle der Sequenzierung von linearer Doppelstrang-DNA, da eine zuverlässige Sequenzierung derartiger Proben nur dann gelingt, wenn ausreichend niedrige DNA-Konzentrationen verwendet werden. Der Einfluß der zur Sequenzierung eingesetzten DNA-Menge auf die Qualität der Sequenzierreaktionen ist aus Beispiel 4 zu ersehen.

Der entscheidende Unterschied zwischen dem erfindungsgemäßen Verfahren und den derzeit üblichen Verfahren der DNA-Sequenzierung nach Sanger liegt in der zyklischen Führung der Sequenzierungsreaktion, wobei eine sehr große Anzahl von Zyklen verwendet wird, und zusätzlich in der Verwendung sehr geringer DNA-Konzentrationen bei extrem hohen Verhältnissen von Primer-Molekülen zu sequenzierenden DNA-Molekülen. Die sehr große Anzahl von Zyklen ermöglicht einerseits bei Verwendung eines großen Überschusses an Oligonukleotidprimer das Erreichen vergleichsweise sehr hohen Fragmentausbeuten. Insbesondere besteht durch die Kombination von sehr geringer DNA-Konzentration und sehr großem Verhältnis zwischen Primer- und DNA-Konzentration die Möglichkeit, sehr kritische DNA-Proben zu sequenzieren, wie zum Beispiel doppelsträngige PCR-Produkte.

### Beispiel 1

### Sequenzierung eines PCR-Produktes

### PCR

0,1 fMol eines in der multi-cloning-site ein 600 bp langes Insert enthaltenden pUC18-Derivates wurden in Anwesenheit von jeweils 4 pMol des (-21)-Standard-Primers (5'-CGT TGT AAA ACG ACG GCC AGT-3') und des Reverse-Primers (5'-CAC ACA GGA AAC AGC TAT GAC-3') sowie in Anwesenheit von je 20 nMol der vier Desoxyribonukleosidtriphosphate in einem Volumen von 100 µl PCR-Puffer (50 mM KCl, 10 mM Tris-HCl, 1,5 mM MgCl₂, 0,01 % Gelatine, pH 8,3) mit 5 units Taq-Polymerase (Cetus) amplifiziert.

Dazu wurde die Reaktionsmischung mit 100 µl Mineralöl überschichtet und in einem programmierbaren Thermostatisierblock (Programmable Dri-Block PHC-1, Techne) 30 Temperaturzyklen bestehend aus 1,0 min. 96°C, 0,5 min. 37°C und 1,0 min. 72°C unterworfen.

Anschließend erfolgte mit Hilfe einer Biospin P30-Säule (Biorad) entsprechend den Herstellerangaben und nach Äquilibrierung der Säule mit dem PCR-Puffer für die wäßrige Phase der Reaktionslösung eine Abtrennung der Desoxyribonukleosidtriphosphate.

### Sequenzierung

Das Eluat der Biospin-Säule wurde ohne weitere Behandlung zur Sequenzierung eingesetzt. Für die vier Sequenzierungsreaktionen wurden folgende Ansätze verwendet.

| | A-Reaktion | C-Reaktion | G-Reaktion | T-Reaktion |
|---|---|---|---|---|
| zu sequenzierendes Produkt | 0,033 pMol | 0,033 pMol | 0,066 pMol | 0,066 pMol |
| Primer * | 0,33 pMol | 0,33 pMol | 0,66 pMol | 0,66 pMol |
| | JOE(-21) | FAM(-21) | TAMRA(-21) | ROX(-21) |
| dATP | 6,25 µM | 25 µM | 50 µM | 50 µM |
| dCTP | 25 µM | 6,25 µM | 50 µM | 50 µM |
| 7-Deaza-dGTP | 37,5 µM | 37,5 µM | 18,8 µM | 75 µM |
| dTTP | 25 µM | 25 µM | 50 µM | 12,5 µM |
| ddATP | 150 µM | - | - | - |
| ddCTP | - | 75 µM | - | - |
| ddGTP | - | - | 25 µM | - |
| ddTTP | - | - | - | 250 µM |
| 10x Puffer** | 5 µl | 5 µl | 5 µl | 5 µl |
| Taq-Polymerase (Cetus) | 2,5 Einheiten | 2,5 Einheiten | 2,5 Einheiten | 2,5 Einheiten |
| Volumen | 50 µl | 50 µl | 50 µl | 50 µl |

| | | | | |
|---|---|---|---|---|
| *) Am 5'-Ende mit Fluoreszenzfarbstoffen markierte (-21)-Standard-Primer: Art.-Nr. 400836 Applied Biosystems | | | | |
| **) 10x Puffer: 10 x PCR-Puffer mit 100 mM MgCl₂ anstelle von 15 mM MgCl₂. | | | | |

Jeder der vier Reaktionsansätze wurde analog der PCR-Reaktion 40 Zyklen bestehend aus 1.0 min. 96°C, 0.5 min. 55°C und 1.0 min. 72°C unterworfen.

Anschließend wurden die wäßrigen Phasen der vier Reaktionsansätze nacheinander auf 20 µl 3 M NaOAc (pH 7.8) pipettiert. Zu dieser Lösung wurden danach 500 µl auf -20°C temperiertes Ethanol gegeben, die Lösung wurde gemischt, 15 min. auf Trockeneis gegeben und 15 min., bei 13000 rpm und RT in einer Eppendorf-Zentrifuge 5415 C zentrifugiert. Das nochmals mit 800 µl 80 % Ethanol gewaschene Pellet wurde danach 2 min unter Vakuum getrocknet, in 2 µl 50 mM EDTA (pH 7.5) und 6 µl deionisiertem Formamid aufgenommen, 2 min auf 95°C erhitzt, in Eiswasser abgekühlt und in einem DNA-Sequencer 370A (Applied Biosystems) einer Elektrophorese (PAG mit 6 % Acrylamid (AA), AA/bis-AA: 40:2; Puffer: 0,089 M Tris, 0,089 M Borat, 2 M EDTA, 7 M Harnstoff, pH 8,3; Leistung: 20 W) unterworfen. Die Steuerung des Sequencers sowie die Auswertung der Elektropherogramme erfolgte mit der Softwareversion 1.30 des Herstellers.

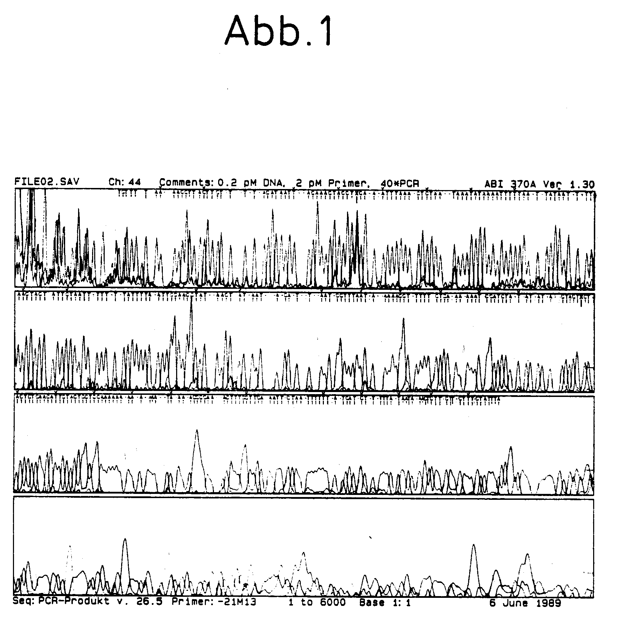

Das erhaltene Ergebnis ist in Abbildung 1 dargestellt. Ein Vergleich zwischen der ermittelten Sequenz und der zuvor schon bekannten Sequenz zeigte, daß mit dem geschilderten Verfahren abgesehen von zwei Problemstellen wenigstens 400 Basen eindeutig ermittelt werden können.

### Beispiel 2

### Sequenzierung von Lambda-DNA

Die Sequenzierung einer Lambda-DNA (Lambda ZAP, Stratagene) wurde analog Beispiel 1 durchgeführt. Es wurden lediglich an Stelle des PCR-Produktes entsprechende Mengen der Lambda-DNA eingesetzt, alle anderen Parameter blieben unverändert.

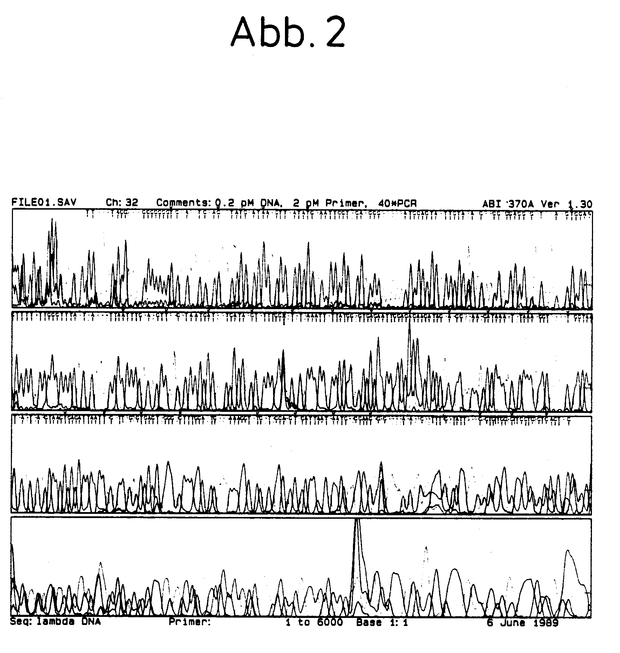

Das Ergebnis ist in Abb. 2 dargestellt. Auch in diesem Fall erlaubte die beschriebene Methode die zuverlässige Ermittelung von wenigstens 400 Basen.

### Beispiel 3

### Sequenzierung eines linearisierten Plasmids

Das Plasmid pUC18 wurde mit der Restriktionsendonuklease XmnI linearisiert und analog Beispiel 1 sequenziert. Abweichend von Beispiel 1 wurden allerdings 0,1 pMol (A- und C-Reaktion) bzw. 0,2 pMol (G- und T-Reaktion) der DNA sowie 2 pMol (A,C) bzw. 4 pMol (G,T) der Primer eingesetzt, und lediglich zehn Temperaturzyklen der Art wie bei der PCR in Beispiel 1 verwendet.

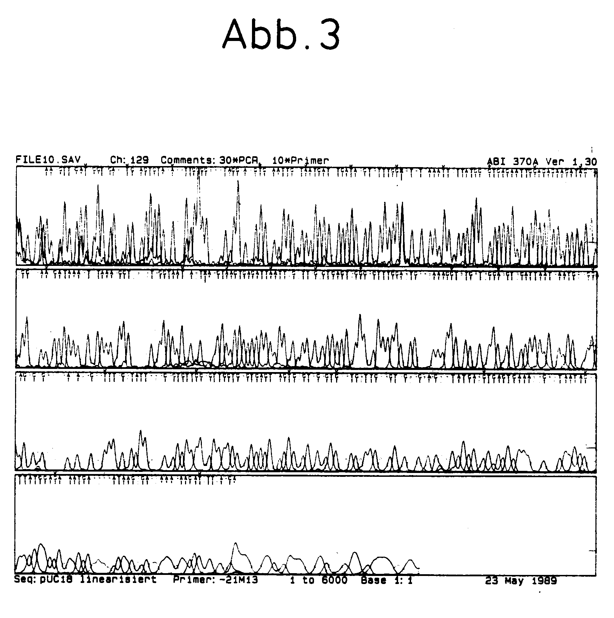

Das Ergebnis ist in Abb. 3 dargestellt. Abgesehen von drei nicht zuordenbaren Basen war bei diesem Experiment ebenso die zuverlässige Bestimmung von wenigstens 400 Basen möglich.

### Beispiel 4

### Sequenzierung eines PCR-Produktes mit unterschiedlichen DNA-Mengen

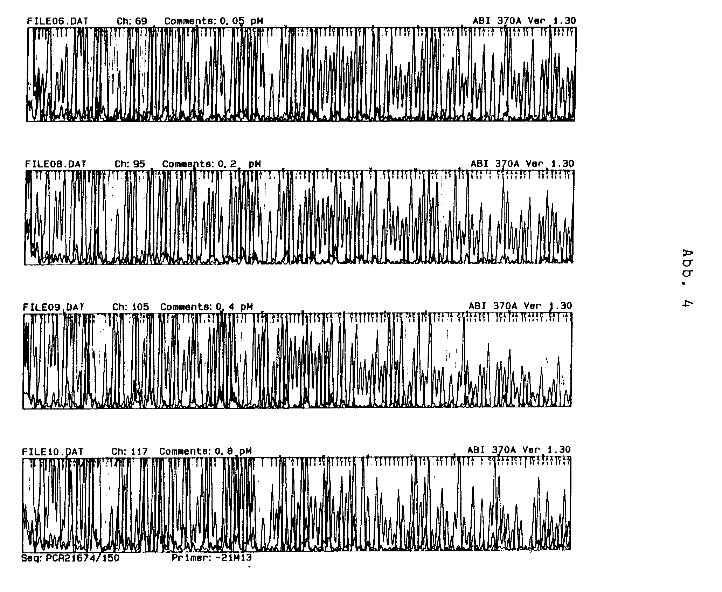

Es wurde ein 1450 Gasen langes PCR-Fragment völlig analog zu Beispiel 1 sequenziert, wobei die Menge der eingesetzten DNA zwischen 0,05 und 0,8 pMol variierte. Die für die ersten etwa 130 Basen erhaltenen Ergebnisse sind in Abb. 4 zusammengefaßt. Es wird deutlich, daß mit zunehmender DNA-Menge eine deutliche Zunahme von unspezifischen Abbrüchen erfolgt.

## Patentansprüche

1. Verfahren zur Sequenzierung von Desoxyribonukleinsäuren, welches darin besteht, daß man die Desoxyribonukleinsäure in einer Konzentration von 10⁻¹⁶ bis 2·10⁻¹⁴ Mol pro µl, im Falle von doppelsträngiger, nicht in der Supercoil-Form vorliegender Desoxyribonukleinsäure vorzugsweise in einer Konzentration von 10⁻¹⁶ bis 3·10⁻¹⁵ Mol pro µl,
a) einem Hitzedenaturierungsschritt
b) einem Annealingschritt und
c) einem Nukleinsäuresyntheseschritt mit teilweisem Kettenabbruch
unterwirft, die Schritte a) bis c) 10- bis 60mal wiederholt und anschließend das erhaltene Desoxyribonukleinsäurefragment-Gemisch der Länge nach auftrennt und aus dem Fragmentspektrum die Sequenz bestimmt.

## Claims

1. A method for deoxyribonucleic acid sequencing, which comprises subjecting the deoxyribonucleic acid in a concentration of from 10⁻¹⁶ to 2·10⁻¹⁴ mol per µl or, in the case of double-stranded, non-supercoiled deoxyribonucleic acid, preferably in a concentration of from 10⁻¹⁶ to 3·10⁻¹⁵ mol per µl to
a) a heat-denaturation step
b) an annealing step and
c) a nucleic acid synthesis step with some chain terminations,
repeating steps a) to c) 10 to 60 times and subsequently fractionating the resulting deoxyribonucleic acid fragment mixture according to length, and determining the sequence from the fragment spectrum.

## Revendications

1. Procédé de séquençage d'acides désoxyribonucléiques, caractérisé en ce que l'on soumet l'acide désoxyribonucléique, en une concentration de 10⁻¹⁶ à 2.10⁻¹⁴ mole par µl, dans le cas d'acide désoxyribonucléique bicaténaire, ne se présentant pas sous la forme Supercoil, de préférence en une concentration de 10⁻¹⁶ à 3.10⁻¹⁵ mole par µl,
a) une étape de dénaturation thermique,
b) une étape d'appariement et
c) une étape de synthèse d'acide nucléique avec rupture partielle de chaîne,
on répète 10 à 60 fois les étapes a) à c) et on sépare ensuite le mélange de fragments d'acide désoxyribonucléique obtenu selon la longueur et on détermine la séquence du spectre des fragments.
